# EUROPEAN PATENT APPLICATION

(11) **EP 2 792 346 A1**
(43) Date of publication of application: **22.10.2014**
(21) Application number: 12856656.9
(22) Date of filing: 12.12.2012
(51) Int. Cl.: A61K 8/81, A61K 8/33, A61K 8/37, A61K 8/73, A61Q 3/02

(54) **MANICURE COMPOSITION**

(30) Priority: 16.12.2011 JP 2011275696; 09.11.2012 JP 2012247661
(71) Applicant: Mitsubishi Pencil Company, Limited, Tokyo 140-8537 (JP)
(72) Inventor: SAKUMA Satoshi, Fujioka-shi Gunma 375-8501 (JP); AZUMA, Mitsuo, Osaka 5810075 (JP); IKEMOTO, Yosuke, Osaka 5810075 (JP)
(74) Representative: Vossius & Partner
(86) International application number: PCT/JP2012/082187
(87) International publication number: WO 2013/089135

(57) **Abstract**

Provided is a manicure composition which is applied as a top coat on a manicure coated on a whole part of a nail and is turned into a transparent coating film after drying and which is top-coated to thereby provide the coating film with a satisfactory lasting property and endows it with glossy cracked patterns. The manicure composition is a cosmetic applied on a dried coating film of a nail cosmetic comprising at least nitrocellulose, acetic acid ester and a plasticizer, and it comprises at least water, an acryl resin emulsion having a glass transition temperature (Tg) of 50°C or higher and 2.5 to 11 % by mass of a film formation aid.

## Description

### Technical Field

The present invention relates to a manicure composition (hereinafter referred to as a top coat) which is applied on a dried coating film of a nail cosmetic comprising at least nitrocellulose, acetic acid ester and a plasticizer.

### Background Art

In general, a top coat is used for enhancing and protecting a gloss of a nail cosmetic, but the purposes thereof are limited. Also, in recent years, products which are applied on a manicure to provide cracked patterns are available, but since they contain particles of silica and the like, a color tone of the cracked patterns are matted, and a top coat has to be further applied thereon in order to provide them with a glossiness.

There have so far been known as top coats and the like applied on a dried coating film or a nail cosmetic comprising at least nitrocellulose, acetic acid ester and a plasticizer, for example:
1) a makeup method and a makeup kit which provide nails with an uneven makeup and/or a discontinuous film and which assume a form of a nail enamel showing an excellent adhesive property with the passage of time, wherein the makeup kit comprises a first composition (a base manicure) which contains a medium allowed to be added as a cosmetic and at least one first film-forming polymer and which forms a film having a hardness d1 falling in a range of 20 to 85 seconds, and a second composition which contains a medium allowed to be added as a cosmetic and at least one second film-forming polymer and which forms a second film having a hardness d2, wherein d2 - d1 is 10 seconds or more (refer to, for example, a patent document 1), and
2) a nail enamel comprising a polymer emulsion and an oxyalkylene glycol derivative having specific physical properties, in which a total content thereof is 1 to 60 % by weight in terms of solid matters, a red pigment, bentonite, a silicone base defoaming agent, an antiseptic agent, and the like (refer to, for example, a patent document 2).

However, in the makeup method and the makeup kit disclosed in the patent 1 described above, it is prescribed that the first film (base manicure) has a hardness d1 of 20 to 85 seconds which is a value measured by means of a pendulum type test machine and that the second film (top coat) has a hardness d2, in which d2 - d1 is 10 seconds or more. That is, it is prescribed that the top coat has to be harder than the base manicure, but the ground thereof is not described therein, and actually, the intended cracks are obtained even when the base manicure is harder than the top coat, or d1 falls outside a range of 20 to 85 seconds. Also, it is described that the top coat can not be formed on a part where the base manicure is not present, but the film thereof is measured by means of the pendulum type test machine, and it is contradictory.

Also, the film-forming resin used has a low glass transition temperature (Tg) and provides a soft film, and the resulting film is soft. Accordingly, the existing situation is that involved therein as well is the problem that the satisfactory lasting property (makeup durability) is not obtained.

Further, cracked patterns are not obtained even by applying the nail enamel (Example 3) described in the patent document 2 described above on a dried coating film of a nail cosmetic comprising at least nitrocellulose, acetic acid ester and a plasticizer.

On the other hand, an enamel composition for manicure and a method for using the same in which after preparing a first liquid containing amorphous silica, magnesium silicate, nitrocellulose and n-butyl acetate and a second liquid containing a coloring matter and n-butyl acetate, the first liquid is mixed with the second liquid to finally prepare a homogeneous composition and in which the composition is controlled to a viscosity suitable for applying it by using n-butyl acetate and then applied on a nail are known as an enamel composition for manicure containing a coloring matter having such a concentration that irregular cracked patterns are formed on a coating film layer when the surface of the composition applied repeatedly on nails of hands and feet is dried (refer to, for example, a patent document 3).

In the above enamel composition for manicure described in the patent document 3, the cracked patterns are obtained, but the existing situation is that involved therein is the problem that due to particles of amorphous silica and magnesium silicate which are contained therein, the patterns are matted in a color tone and are not endowed with a gloss (a digital variable angle gloss meter: a gloss value measured at 45° in UGV-5D is about 10).

### Prior Art Documents

### Patent documents

Patent document 1: JP 2000-109406 A (claims, examples and the like)
Patent document 2: JP Hei 6-56624 A (claims, Example 3)
Patent document 3: U.S Patent No. 5935590 (claims, examples, Fig. 1 and the like)

### Outline of the Invention

### Problems to be solved by the Invention

In light of the foregoing problems and existing situations of the conventional technologies, the present invention intends to solve them, and an object thereof is to provide a manicure composition which is used for a top coat applied on a dried coating film of a nail cosmetic (base manicure) comprising at least nitrocellulose, acetic acid ester and a plasticizer and coated on a whole part of a nail, wherein the manicure composition top-coated thereon is turned into a transparent coating film after dried, and the coating film is endowed with a satisfactory makeup durability and provided with glossy cracked patterns.

### Means for Solving the Problems

In light of the foregoing problems of the conventional technologies, the present inventors intend to solve them and have found that a manicure composition which meets the object described above is obtained by containing at least water, a resin having no film formation property at normal temperature and a film formation aid which dissolves a base manicure and can cause the above resin to form an even film at normal temperature, and thus they have come to complete the present invention.

That is, the present invention comprises the following items (1) to (4).
(1) A manicure composition which is a cosmetic applied on a dried coating film of a nail cosmetic comprising at least nitrocellulose, acetic acid ester and a plasticizer and which comprises at least water, an acryl resin emulsion having a glass transition temperature (Tg) of 50°C or higher and 2.5 to 11 % by mass of a film formation aid.
(2) A manicure composition which is a cosmetic applied on a dried coating film of a nail cosmetic comprising at least nitrocellulose, acetic acid ester and a plasticizer and which comprises at least water, an acryl resin emulsion having a glass transition temperature (Tg) of 50°C or higher and 2.5 to 11 % by mass of a film formation aid, wherein a coating film of the cosmetic applied is cracked in drying.
(3) The manicure composition as described in the above item (1) or (2), wherein the film formation aid is diethylene glycol diethyl ether and/or propylene carbonate.
(4) The manicure composition as described in any one of the above items (1) to (3), further comprising at least one of ethanol, isopropyl alcohol and acetone.

### Effects of the Invention

According to the present invention, provided is a manicure composition which is top-coated on a manicure to thereby provide cracked patterns having a high gloss and a satisfactory makeup durability.

### Mode for Carrying out the Invention

The embodiments of the present invention shall be explained below in detail.

The manicure composition of the present invention is characterized by that it is a cosmetic applied on a dried coating film of a nail cosmetic comprising at least nitrocellulose, acetic acid ester and a plasticizer and that it comprises at least water, an acryl resin emulsion having a glass transition temperature (Tg) of 50°C or higher and 2.5 to 11 % by mass of a film formation aid.

The acryl resin emulsion used in the present invention is a resin which does not have a film formation property at normal temperature (25°C; hereinafter the same shall apply), and the above resin can form an even film at normal temperature by virtue of a film formation aid described later.

The acryl resin emulsion used is provided with a glass transition temperature (Tg) of 50°C or higher, preferably 60°C or higher from the viewpoints of forming a hard film after dried and securing a satisfactory lasting property. An upper limit of Tg shall not specifically be restricted, but if it exceeds 100°C, an adhesive property thereof with the base manicure is not obtained, and therefore it is preferably 100°C or lower.

The glass transition temperature (Tg) was measured in the following manner. First, the resin was turned into a lacquer, and a film was formed from the lacquer. Then, the solvent was removed from the film to thereby dry the film. The base acryl resin turned into the film was heated from -20°C up to +150C at a heating rate of 10°C/minute by means of a differential scanning calorimeter (DSC) manufactured by Rigaku Corporation to measure a differential scanning calorie. The endothermic curve thus obtained was differentiated to determine an inflection point thereof, and the above inflection point was judged as Tg.

The usable acryl resin emulsion includes emulsions having the characteristics described above, such as alkyl acrylate copolymer emulsions, acrylic acid ester-methacrylic acid ester copolymer emulsions, alkyl acrylate-styrene copolymer emulsions and the like. From the viewpoints of a hardness and a gloss, the alkyl acrylate-styrene copolymer emulsions are preferably used.

SE-2696F (manufactured by Taisei Fine Chemical Co., Ltd.) can be listed as the specific alkyl acrylate copolymer emulsions, and JONCRYL 352, 538J, 7640, 7641, 631, 790, 780 and 7610 (manufactured by BASF A.G.), and SE-810A, SE-841A, SE-953A-2 and SE-1658F (manufactured by Taisei Fine Chemical Co., Ltd.) can be listed as the alkyl acrylate-styrene copolymer emulsions. They can be used at least in a single kind (each alone or in a mixture of two or more kinds thereof).

A content of the acryl resin emulsions having the above characteristics is 10 to 50 % by mass (hereinafter referred to merely as "%") in terms of a solid content, preferably 15 to 40 % based on a whole amount of the manicure composition.

If a content of the above acryl resin emulsions is less than 10 %, the coating film is thin, and the lasting property becomes unsatisfactory. Accordingly, it is not preferred. On the other hand, if it exceeds 50 %, it is difficult to blend other components such as the film formation aid and the like, and the physical properties are varied to a large extent due to vaporization of water in storing and using. Accordingly, it is not preferred as well.

The film formation aid used in the present invention shall not specifically be restricted as long as it dissolves a dried coating film of the base manicure comprising at least nitrocellulose, acetic acid ester and a plasticizer and has a function of making it possible to form an even film at normal temperature from the foregoing acryl resin having no film formation property at normal temperature, and from the viewpoints of a usability, a safety and the like, it includes at least one kind of diethylene glycol diethyl ether, propylene carbonate, diethylene glycol monobutyl ether, propylene glycol monomethyl ether (PGM), diethylene glycol monoethyl ether, and the like.

From the viewpoints of a balance between a solubility of the base manicure and a film forming function of the acryl resin at normal temperature, diethylene glycol diethyl ether and/or propylene carbonate are preferably used.

A content of the above film formation aids is 2.5 to 11 %, preferably 3 to 10 % based on a whole amount of the manicure composition.

If a content of the above film formation aid is less than 2.5 %, the film formation aid can not dissolve the base manicure, and an adhesive property between the base manicure and the top coat is unsatisfactory. Accordingly, it is not preferred. On the other hand, if it exceeds 11 %, a film of the top coat is formed, and the cracked patterns are not obtained. Accordingly, it is not preferred as well.

From the viewpoint of a drying property and an antiseptic and antifungal property, at least one solvent selected from ethanol, isopropyl alcohol and acetone in addition to the respective components described above is preferably added to the manicure composition of the present invention, and at least one selected from ethanol, isopropyl alcohol and acetone is more preferably added thereto.

A content of the above solvents is 3 to 50 %, preferably 10 to 40 % based on a whole amount of the manicure composition.

If a content of the above solvent is less than 3 %, the drying property and the antiseptic and antifungal property are unsatisfactory, and it is not preferred. On the other hand, if it exceeds 50 %, a stability of the emulsion is unsatisfactory, and it is not preferred as well.

Also, the aqueous manicure composition of the present invention is adjusted by water (refined water, distilled water, ion exchanged water, purified water and the like) as the balance, and in addition to the respective components described above, optional components such as a pH controlling agent, a water-soluble organic solvent, an antiseptic and antifungal agent, a defoaming agent, a viscosity controlling agent, and the like can suitably be added as long as the effects of the present invention are not damaged.

Also, a color stone nail manicure which provides cracked color patterns may be prepared by further adding a coloring material to the aqueous manicure composition of the present invention comprising the respective components described above.

Pigments, dyes and the like can be listed as the coloring material which can be used in the present invention.

The pigments which can be used in the present invention shall not specifically be restricted as long as they are usually used as pigments for liquid cosmetics, and inorganic pigments and organic pigments can be used.

It includes, for example, at least one selected from inorganic pigments such as carbon blacks, black iron oxide, yellow iron oxide, chromium oxide, ultramarine, Prussian blue, zinc oxide, aluminum oxide, silicon dioxide, titanium oxide, magnesium oxide, chromium hydroxide, calcium carbonate, titan yellow, colcothar, and the like, pearl pigments (highly brilliant particles) such as titanium dioxide-coated scaly titanium, colcothar-coated mica titanium, fish scale guanines, N- ε-1-auroyl-L-lysine-coated talc, and the like, highly brilliant pigments such as laminated film powders and the like, and organic pigments such as Blue No. 1 Al lake, Red No. 202, Red No. 220, Red No. 226, Red No. 228, Blue No. 201, Blue No. 204, Blue No. 404, Yellow No. 401, Yellow No. 205, Yellow No. 4 Al lake, Yellow No. 203 Al lake, Red No. 104 Al lake, and the like.

A dispersant which is used, if necessary, when the pigments described above are used is selected according to a compatibility with the pigments used, and acryl base resins and surfactants are used therefor and shall not specifically be restricted.

The dyes which can be used in the present invention shall not specifically be restricted as long as they are usually used as dyes for liquid cosmetics.

The above dyes are preferably, for example, dyes which have so far been used for cosmetics and the like and which are highly safe to human bodies, and they include, for example, nitro dyes, azo dyes, nitroso dyes, triphenylmethane dyes, xanthene dyes, quinoline dyes, anthraquinone dyes, indigo dyes, and the like. To be specific, capable of being listed from the viewpoint of the dyeing power are, for example, Red No. 2, Red No. 3 (FD & C Red No. 3), Red No. 40 (FD & C Red No. 40), Red No. 102, Red No. 104 (D & C Red No. 28), Red No. 105, Red No. 106, Red No. 201 (D & C Red No. 6), Red No. 202 (D & C Red No. 7), Red No. 203, Red No. 205, Red No. 227 (D & C Red No. 33), Red No. 230-1 (D & C Red No. 22), Red No. 401, Red No. 402, Red No. 504 (FD & C Red No. 4), Orange No. 205 (D & C Orange No. 4), Orange No. 402, Yellow No. 4 (FD & C Yellow No. 5), Yellow No. 5 (FD & C Yellow No. 6), Yellow No. 203 (D & C Yellow No. 10), Yellow No. 402, Yellow No. 403-1 (Ext. D & C Yellow No. 7), Yellow No. 406, Yellow No. 407, Green No. 3 (FD & C Green No. 3), Green No. 201, Green No. 402, Blue No. 1 (FD & C Blue No. 1), Blue No. 2 (FD & C Blue No. 2), Blue No. 203, Blue No. 205 (D & C Blue No. 4), Blue No. 403, Blue No. 404, Brown No. 201 (D & C Brown No. 1), Violet No. 401 (Ext. D & C Violet No. 2), Black No. 401, and the like. At least one selected from the above dyes can be used and shall not specifically be restricted as long as it is a dye used for liquid cosmetics.

When the dye is used, a UV absorber can be used, if necessary, in a suitable amount in order to inhibit the fading caused by light.

The UV absorber includes, for example, at least one selected from benzophenone derivatives such as 2-hydroxy-4-methoxybenzophenone, 2-hydroxy-4-methoxybenzophenone-5-sulfonic acid, sodium 2-hydroxy-4-methoxybenzophenone-5-sulfonate, dihydroxymethoxybenzophenone, sodium dihydroxymethoxybenzophenonesulfonate, 2,4-dihydroxybenzophenone, tetrahydroxybenzophenone, and the like, paraaminobenzoic acid derivatives such as paraaminobenzoic acid, ethyl paraaminobenzoate, glyceryl paraaminobenzoate, amyl paradimethylaminobenzoate, octyl paradimethylaminobenzoate, and the like, methoxycinnamic acid derivatives such as ethyl paramethoxycinnamate, isopropyl paramethoxycinnamate, octyl paramethoxycinnamate, 2-ethoxyethyl paramethoxycinnamate, sodium paramethoxycinnamate, potassium paramethoxycinnamate, glyceryl paramethoxycinnamate mono-2-ethylhexanoate, and the like, salicylic acid derivatives such as octyl salicylate, phenyl salicylate, homomenthyl salicylate, dipropylene glycol salicylate, ethylene glycol salicylate, myristyl salicylate, methyl salicylate, and the like, urocanic acid, ethyl urocanate, 4-tert-butyl-4'-methoxydibenzoylmethane, 2-(2'-hydroxy-5'-methylphenyl)benzotriazole, methyl anthranilate, fine particle titanium oxide, fine particle zinc oxide, and zirconium.

A total content of the coloring materials such as the above pigments and dyes is preferably 0.005 to 10 %, more preferably 0.01 to 5 % based on a whole amount of the manicure composition from the viewpoints of obtaining cracked color patterns and enhancing the ageing stability, the coating performance, the high gloss, the color developability, and the like.

The manicure composition of the present invention is prepared by mixing and dispersing the respective components described above at contents falling in the respective ranges described above by means of a mix disperser, for example, a bead mill, a homomixer, a disper, an attritor, and the like.

The present invention relates to, as described above, the manicure composition applied on a dried coating film of a base manicure, and the composition for obtaining the intended dried coating film of the base manicure shall not specifically be restricted as long as it comprises at least nitrocellulose, acetic acid ester and a plasticizer. It is preferably a dried coating film of a base manicure comprising at least nitrocellulose, acetic acid ester, an alkyl acrylate copolymer, a pigment and a plasticizer.

The use form of the manicure composition of the present invention includes, for example, a manicure applicator constituted from a bottle (vessel) in which the above manicure composition is received and a brush as a coating member equipped in a cap, and a manicure applicator comprising at least a liquid receiving space for receiving the manicure composition and a coating member of a so-called pen feed type prepared by solidifying fibers with a resin or the like or prepared by fusing fibers, wherein the manicure composition is delivered from the liquid receiving space to the coating member described above by virtue of a capillary force, and the manicure composition received in the liquid receiving space of the applicator can suitably be used.

The manicure composition of the present invention constituted in the manner described above is top-coated on a dried coating film of the nail cosmetic (base manicure) comprising at least nitrocellulose, acetic acid ester and a plasticizer, whereby the acryl resin which does not form a film at normal temperature is turned partially into a film by the action of the film formation aid. Also, the dried coating film of the base manicure is dissolved, whereby the coating film is cracked in drying, and beautiful cracked patterns are obtained. Further, the acryl resin used is endowed with a high Tg, whereby obtained is the manicure composition providing a coating film which is hard in drying and provided with a satisfactory manicure durability (lasting property) and which is endowed with a high gloss (a gloss value measured at 45° by means of a digital variable angle gloss meter is 30 or more).

### Examples

Next, the present invention shall be explained in further details with reference to examples and comparative examples, but the present invention shall not be restricted by the following examples.

### [Examples 1 to 9 and Comparative Examples 1 to 6]

The components were mixed and dispersed in blend compositions shown in the following Table 1 by means of a homomixer or a disper to prepare the respective manicure compositions.

The respective manicure compositions obtained in Examples 1 to 9 and Comparative Examples 1 to 6 described above were used to evaluate a coating film state (presence of cracked patterns), a coating film state (presence of a gloss) and a manicure durability (lasting property) by the following methods. The results thereof are shown in the following Table 1.

### [Evaluation methods for a coating film state (presence of cracked patterns) and a coating film state (presence of a gloss)]

A commercial manicure (base manicure) having the following composition was applied on nails of both hands and left standing for 15 minutes after applying, and then the respective manicure compositions were applied on the dried coating films of the base manicure by means of a commercial nail brush to visually confirm the presence of cracked patterns and evaluate them functionally according to the following evaluation criteria. Further, the presence of a gloss including cracked patterns was confirmed by measuring a gloss value at 45° by means of a digital variable angle gloss meter (UGV-5D manufactured by Suga Test Instruments Co., Ltd.) to evaluate it according to the following evaluation criteria.

| | |
|---|---|
| (Manicure composition, whole amount 100 %) | |
| Nitrocellulose | 12 % |
| Ethyl acetate | 30 % |
| Butyl acetate | 40 % |
| Plasticizer | 4 % |
| (acetyl triethyl citrate) | |
| Alkyl acrylate copolymer | 5 % |
| Other optional components | balance |

### Evaluation criteria of a coating film state (presence of cracked patterns):

○: cracked patterns were provided
Δ: cracked patterns were partially provided
×: cracked patterns were not provided

### Evaluation criteria of a coating film state (presence of a gloss):

○: high gloss (gloss value: 30 or more)
Δ: a little inferior gloss (gloss value: 15 or more to less than 30)
×: whitened and not glossy (gloss value: less than 15)

### [Evaluation method for a makeup durability (lasting property)]

The coating film states were observed by 5 panelists after two days since the manicure compositions were applied, and they were evaluated according to the following evaluation criteria.
Evaluation criteria:
○: scarcely different from before use
Δ: a little peeled at a tip of a nail
×: peeled to a large extent and reduced in a gloss

**Table 1**

| | | Example | | | | | | | | | Comparative Example | | | | | |
|---|---|---|---|---|---|---|---|---|---|---|---|---|---|---|---|---|
| | | 1 | 2 | 3 | 4 | 5 | 6 | 7 | 8 | 9 | 1 | 2 | 3 | 4 | 5 | 6 |
| | Acryl resin emulsion A*1 | 60 | 60 | 60 | 60 | 60 | 60 | 80 | 60 | 60 | 60 | 60 | | | | |
| | Diethylene glycol diethyl ether | 4 | 8 | | | | 4 | 4 | 4 | 4 | 2 | 12 | 4 | 4 | 4 | 4 |
| | Propylene carbonate | | | 5 | | | | | | | | | | | | |
| | Diethylene glycol monobutyl ether | | | | 10 | | | | | | | | | | | |
| | Propylene glycol monomethyl ether | | | | | 5 | | | | | | | | | | |
| | Ethanol | 20 | 20 | 20 | 20 | 20 | 20 | 30 | 20 | 20 | 20 | 20 | | | | |
| | Smectite | | | | | | 0.2 | | | | | | | | | |
| | Acryl resin emulsion B*2 | | | | | | | | | | | | 80 | | | |
| Blend composition | Acryl resin emulsion C*3 | | | | | | | | | | | | | 80 | | |
| | Acryl resin emulsion D*4 | | | | | | | | | | | | | | 80 | |
| | Acryl resin emulsion E*5 | | | | | | | | | | | | | | | 80 |
| | Yellow No. 4 | | | | | | | | 0.2 | | | | | | | |
| | 10 % carbon black dispersion* 6 | | | | | | | | | 5 | | | | | | |
| | Fine particle titanium oxide | | | | | | | | 0.02 | | | | | | | |
| | Refined water | Balance | Balance | Balance | Balance | Balance | Balance | Balance | Balance | Balance | Balance | Balance | Balance | Balance | Balance | Balance |
| Evaluation | Coating film state (presence of cracked patterns | ○ | ○ | ○ | ○ | Δ | ○ | ○ | ○ | ○ | ○ | × | × | × | × | × |
| | Coating film state (presence of gloss) | ○ | ○ | ○ | ○ | Δ | ○ | ○ | ○ | ○ | × | ○ | ○ | ○ | ○ | ○ |
| | Gloss value | 40 | 45 | 39 | 45 | 18 | 37 | 39 | 40 | 40 | 5 | 90 | 80 | 60 | 71 | 95 |
| | Makeup durability (lasting property) | ○ | ○ | ○ | Δ | Δ | ○ | ○ | ○ | ○ | × | ○ | Δ | × | Δ | Δ |

| | | | | | | | | | | | | | | | | |
|---|---|---|---|---|---|---|---|---|---|---|---|---|---|---|---|---|
| *1: alkyl acrylate-styrene copolymer (Tg: 80°C, solid content:40 %) *2: alkyl acrylate copolymer (ET-410, Tg: 44°C, solid content:30 %, manufactured by Toagosei Co., Ltd.) *3: alkyl acrylate copolymer (GH800F, Tg: -15°C, solid content:45 %, manufactured by Akzo Nobel N.V.) *4: alkyl acrylate copolymer (GH810F, Tg: 20°C, solid content:46 %, manufactured by Akzo Nobel N.V.) *5: alkyl acrylate-styrene copolymer (Joncryl 775, Tg: 37°C, solid content:45 %, manufactured by BASF A.G.) *6: dispersant AMPHOMER containing 2 % of HC | | | | | | | | | | | | | | | | |

As apparent from the results shown in Table 1 described above, it has been found that the manicure compositions prepared in Examples 1 to 9 falling in the scope of the present invention are applied on a base manicure coated on a whole part of a nail to be turned into a transparent top coat as compared with the manicure compositions prepared in Comparative Examples 1 to 6 falling outside the scope of the present invention. Further, it has been confirmed that the above manicure compositions are top-coated, whereby the coating films are provided with a satisfactory makeup durability (lasting property) and a high gloss and that the coating films are cracked in drying to be provided with beautiful cracked patterns.

### Industrial Applicability

Provided is a manicure composition of a top coat type endowed with a nail art.

## Claims

1. A manicure composition which is a cosmetic applied on a dried coating film of a nail cosmetic comprising at least nitrocellulose, acetic acid ester and a plasticizer and which comprises at least water, an acryl resin emulsion having a glass transition temperature (Tg) of 50°C or higher and 2.5 to 11 % by mass of a film formation aid.

2. A manicure composition which is a cosmetic applied on a dried coating film of a nail cosmetic comprising at least nitrocellulose, acetic acid ester and a plasticizer and which contains at least water, an acryl resin emulsion having a glass transition temperature (Tg) of 50°C or higher and 2.5 to 11 % by mass of a film formation aid, wherein a coating film of the cosmetic applied on the dried coating film is cracked in drying.

3. The manicure composition as described in claim 1 or 2, wherein the film formation aid is diethylene glycol diethyl ether and/or propylene carbonate.

4. The manicure composition as described in any one of claims 1 to 3, further comprising at least one of ethanol, isopropyl alcohol and acetone.
